# EUROPÄISCHE PATENTANMELDUNG

(11) **EP 0 617 003 A1**
(43) Veröffentlichungstag der Anmeldung: **28.09.1994**
(21) Anmeldenummer: 94104281.4
(22) Anmeldetag: 18.03.1994
(51) Int. Cl.: C07C 67/36, C07C 69/06

(54) **Verfahren zur Herstellung von Methylformiat**

(30) Priorität: 25.03.1993 DE 4309731
(71) Anmelder: BASF Aktiengesellschaft, D-67063 Ludwigshafen (DE)
(72) Erfinder: Zehner, Peter, Dr., D-67071 Ludwigshafen (DE); Bittins, Klaus, Dr., D-67227 Frankenthal (DE); Haarde, Wilhelm, Dr., D-67269 Gruenstadt (DE); Eiden, Ulrich, Dr., D-67227 Frankenthal (DE); Wolff, Dietrich, Dr., D-68723 Plankstadt (DE); Herr, Manfred, D-67157 Wachenheim (DE); Hupfer, Leopold, Dr., D67159 Friedelsheim (DE)

(57) **Zusammenfassung**

Herstellung von Methylformiat durch Umsetzung von Kohlenmonoxid und Methanol unter erhöhtem Druck und erhöhter Temperatur in Gegenwart eines Alkalimetallmethylats, indem man
A) die Ausgangsstoffe in einer Mischzone vermischt, teilweise abreagieren läßt, die Reaktionslösung mit CO sättigt und
B) die Umsetzung in einer oder mehreren Nachreaktionszonen ohne Zufuhr weiterer Ausgangsverbindungen zu Ende führt.

## Beschreibung

Die vorliegende Erfindung betrifft ein Verfahren zur Herstellung von Methylformiat aus Kohlenmonoxid und Methanol unter erhöhtem Druck und erhöhter Temperatur in Gegenwart eines Alkalimetallmethylats.

Diese Herstellweise für Methylformiat ist im Prinzip lange bekannt. Dennoch ergeben sich bei der großtechnischen Umsetzung einige Probleme.

Die US-A 4 661 624 beschreibt ein Verfahren zur Herstellung von Methylformiat aus CO und Methanol in Gegenwart von Natriummethylat als Katalysator. Um eine hohe Reaktionsgeschwindigkeit zu erzielen, wird hier bei hoher Katalysatorkonzentration mit niedrigen Umsätzen gearbeitet. Dies hat zur Folge, daß nicht umgesetztes Methanol nach dem Abdestillieren des Produkts zurückgeführt werden muß, um den Prozeß wirtschaftlich zu gestalten und daß ein Großteil des eingesetzten Kohlenmonoxids nicht genutzt wird oder energieaufwendig nach Kompression zurückgeführt werden muß. Zur besseren CO-Nutzung können in einer bevorzugten Ausführungsform zwei Reaktionszonen hintereinandergeschaltet werden, die beide mit zurückgeführtem Methanol beschickt werden und nacheinander vom CO-Gas durchströmt werden. Der Umsatz von CO bleibt aber unter 90 %.

Die JP-A 87/22744 betrifft ein Verfahren zur Herstellung von Methylformiat aus CO und Methanol in einem ringförmigen Reaktor. Die intensiv vermischten Ausgangsmaterialien werden durch den Reaktor gepumpt. Zur Aufarbeitung gelangt nach der Lehre dieser Schrift ein Gemisch aus Methylformiat, Methanol sowie darin dispergiertem CO, so daß ohne eine entsprechende Rückführung des Gases die Verluste an CO relativ groß sind.

Die DE-A 27 10 726 lehrt ein Verfahren zur Herstellung von Methylformiat, in dem CO durch einen rückgeführten Strom des Reaktionsgemisches in die intensiv durchmischte Reaktionszone gesaugt wird. Ein vollständiger CO-Umsatz ist in diesem Verfahren jedoch nicht zu erreichen, da mindestens diejenige CO-Menge mit dem Reaktionsaustrag ausgeschleust wird, die entsprechend dem CO-Partialdruck hierin gelöst ist.

Es bestand die Aufgabe, ein Verfahren bereitzustellen, das es erlaubt, bei hohem CO-Umsatz und entsprechend geringer CO-Rückführungsrate Methylformiat herzustellen.

Demgemäß wurde ein Verfahren zur Herstellung von Methylformiat aus Kohlenmonoxid CO und Methanol unter erhöhtem Druck und erhöhter Temperatur in Gegenwart eines Alkalimetallmethylats gefunden, das dadurch gekennzeichnet ist, daß man
A) die Ausgangsstoffe in einer Mischzone vermischt, teilweise abreagieren läßt, die Reaktionslösung mit CO sättigt und
B) die Umsetzung in einer oder mehreren Nachreaktionszonen ohne Zufuhr weiterer Ausgangsverbindungen zu Ende führt.

Die Gesamtreaktion läßt sich folgendermaßen wiedergeben: 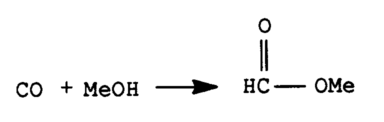
Me = Methyl
Für das erfindungsgemäße Verfahren kann CO, das gegebenenfalls noch ein inertes Gas enthält, beispielsweise Stickstoff, eingesetzt werden, wobei dabei ein Gas mit einem CO-Gehalt von über 93 Vol.-% bevorzugt wird. Im allgemeinen beträgt der Wassergehalt des Gases weniger als 100 ppm, um die Zersetzung des Katalysators zu verhindern. Als Katalysator dienen Alkalimetallmethylate, von denen Natriummethylat bevorzugt wird. CO, Methanol sowie der üblicherweise im Methanol gelöste Katalysator werden in der Mischzone vermischt. Dazu ist es vorteilhaft, die Gasphase in der Flüssigphase möglichst gut zu dispergieren, um dadurch eine schnelle Reaktion zu bewirken.

In der Mischzone führt ein hoher CO-Partialdruck zu einer hohen Reaktionsgeschwindigkeit. Es können während der Reaktion Gesamtdrücke von 10 bis 300 bar herrschen, vorzugsweise 20 bis 150 und besonders bevorzugt 40 bis 100 bar.

Die Temperatur kann 60 bis 120, vorzugsweise 70 bis 90 °C betragen. Die Katalysatorkonzentration beträgt im allgemeinen 0,1 bis 3, vorzugsweise 0,4 bis 1,5 Gew.-%, bezogen auf eingesetztes Methanol. Die molaren Verhältnisse von Methanol und CO können von 2:1 bis 8:1 betragen, bevorzugt werden 3:1 bis 5,5:1, besonders bevorzugt 3,5:1 bis 4,5:1. Ein Überschuß an Methanol erleichtert zum einen einen hohen CO-Umsatz, zum anderen bewirkt er, daß der in reinem Methylformiat unlösliche Katalysator in Lösung bleibt. In der Mischzone kann der CO-Umsatz über die Verweilzeit gesteuert werden; bewährt haben sich hier Umsätze von 85 bis 95 % des eingesetzten CO. Während der Mischphase sättigt sich die Reaktionslösung mit nicht abreagiertem CO.

Das so erhaltene Reaktionsgemisch aus Methylformiat, Methanol, gelöstem Katalysator und gelöstem CO wird erfindungsgemäß in eine oder mehrere Nachreaktionszonen überführt, wobei keine weiteren Ausgangsverbindungen zugeführt werden. Nicht gelöstes CO verbleibt dabei in der Mischzone und das gelöste CO reagiert zum Produkt ab. Bei hinreichender Verweilzeit läuft die Reaktion somit bis zur Gleichgewichtseinstellung ab.

Praktisch realisieren läßt sich diese Nachreaktion z.B. durch die Überführung des Reaktionsgemisches in einen oder mehrere hintereinander angeordnete Kessel, in denen unterschiedliche Konzentrationen herrschen. Es ist auch möglich, die Nachreaktion in einem Rohr ablaufen zu lassen.

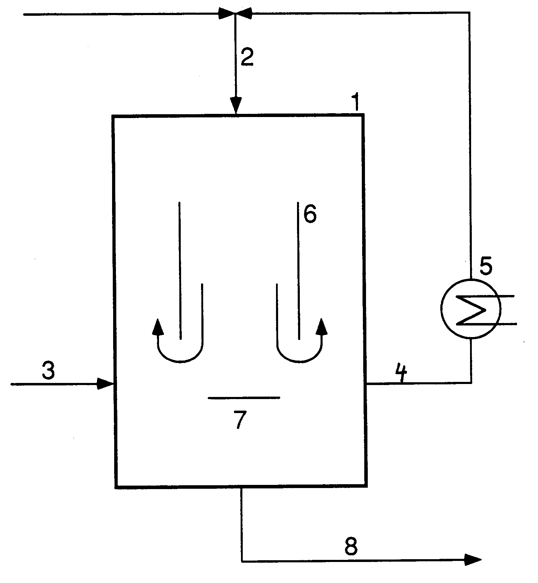

Eine besonders bevorzugte Ausführungsform des erfindungsgemäßen Verfahrens ist in der Figur wiedergegeben. In einen Reaktor 1 werden über eine Zuleitung 2 Methanol und Katalysator gegeben; über eine Zuleitung 3 wird CO über einen Gasverteiler in den Reaktor 1 gegeben. Das Reaktionsgemisch wird über einen äußeren Kreislauf 4 im Kreis geführt. Dabei wird die Reaktionswärme über einen Wärmetauscher 5 abgeführt. Im Reaktor 1 sorgen Führungsvorrichtungen 6 und eine Prallplatte 7 dafür, daß das Reaktionsgemisch in der Mischzone in einen inneren Kreislauf gezwungen wird, wodurch es zu einer innigen Durchmischung von Gas und Flüssigkeit kommt. Die Nachreaktionszone ist unter der Mischzone angeordnet, so daß die Reaktionslösung absinkt. Nicht gelöstes CO kann nach oben in die Mischzone entweichen. Unten am Reaktor 1 kann die ausreagierte Reaktionslösung über eine Leitung 8 entnommen werden.

Die Aufarbeitung der erfindungsgemäß erhaltenen Reaktionslösung erfolgt in an sich bekannter Weise, wobei sich im allgemeinen an eine Entspannung und Restgasabtrennung eine Destillation der flüssigen Komponenten mit eventueller Rückführung des so erhaltenen Methanols anschließt. Methylformiat kann dann in bekannter Weise zu Ameisensäure hydrolysiert werden.

Das erfindungsgemäße Verfahren bietet die Möglichkeit, im Reaktionsgemisch gelöstes CO zu einem solchen Grad umzusetzen, daß eine Rückführung in die Reaktion nicht mehr lohnend ist oder daß sie sich erübrigt. Weiterhin bietet das Verfahren den Vorteil, bei kleinen Reaktorabmessungen eine hohe Raum-Zeit-Ausbeute zu erreichen.

### Beispiel

In einem kontinuierlich betriebenen Reaktor gemäß Figur wurden bei einem Gesamtdruck von 57 bar und bei 80°C Methanol und reines CO im molaren Verhältnis von 3,6:1 in Gegenwart von 1 Gew.-% Natriummethylat, bezogen auf Methanol, vermischt. Bei einer Verweilzeit von 45 Minuten betrug der Methylformiat-Gewichtsanteil 40 %. Der CO-Umsatz betrug 92 %. Durch eine Nachreaktionsphase von 2 Minuten stieg der CO-Umsatz auf 95,5 %, entsprechend halbierte sich fast der CO-Verlust durch im Reaktionsgemisch gelöstes CO.

## Patentansprüche

1. Verfahren zur Herstellung von Methylformiat durch Umsetzung von Kohlenmonoxid und Methanol unter erhöhtem Druck und erhöhter Temperatur in Gegenwart eines Alkalimetallmethylats, dadurch gekennzeichnet, daß man
A) die Ausgangsstoffe in einer Mischzone vermischt, teilweise abreagieren läßt, die Reaktionslösung mit CO sättigt und
B) die Umsetzung in einer oder mehreren Nachreaktionszonen ohne Zufuhr weiterer Ausgangsverbindungen zu Ende führt.

2. Verfahren nach Anspruch 1, dadurch gekennzeichnet, daß man bei einem Druck von 40 bis 100 bar arbeitet.

3. Verfahren nach Anspruch 1 oder 2, dadurch gekennzeichnet, daß man bei einer Temperatur von 60 bis 100°C arbeitet.

4. Verfahren nach den Ansprüchen 1 bis 3, dadurch gekennzeichnet, daß man Natriummethylat als Katalysator verwendet.

5. Verfahren nach den Ansprüchen 1 bis 4, dadurch gekennzeichnet, daß die Katalysatorkonzentration in der Mischzone 0,4 bis 1,5 Gew.-%, bezogen auf eingesetztes Methanol beträgt.
